# EUROPÄISCHE PATENTANMELDUNG

(11) **EP 2 835 644 A1**
(43) Veröffentlichungstag der Anmeldung: **11.02.2015**
(21) Anmeldenummer: 14179889.2
(22) Anmeldetag: 05.08.2014
(51) Int. Cl.: G01N 33/543, A61K 9/127, G01N 33/553, G01N 33/546, A61K 39/395, C07K 16/30, A61K 9/16

(54) **Nanocomposites zur Einkapselung von Zellen und Verfahren zur Behandlung von Krankheiten**

(30) Priorität: 06.08.2013 DE 102013108453
(71) Anmelder: Ussembayev, Yerzhan, 2628 BA Delft (NL)
(72) Erfinder: Ussembayev, Yerzhan, 2628 BA Delft (NL)
(74) Vertreter: Jones Day

(57) **Zusammenfassung**

Die vorliegende Erfindung betrifft einen Polymer-Partikel-Komplex, der für die Einkapselung von lebenden Zellen verwendbar ist. Insbesondere werden (lebende) Zellen mit dem erfindungsgemäßen Polymer-Partikel-Komplex eingekapselt, wobei dabei Krankheiten spezifisch behandelt werden können.

## Beschreibung

Die vorliegende Erfindung betrifft die Einkapselung von lebenden Zellen. Insbesondere werden die Zellen mit Polymer-Partikel-Komplexen eingekapselt, wobei dabei Krankheiten spezifisch behandelt werden können.

Nanotechnologie als ein neuer Trend der modernen Wissenschaft ist in der letzten Zeit rasend schnell in alle Bereiche des täglichen Lebens, einschließlich Industrie, Technologie und Biomedizin, vorgedrungen. Besonderes Interesse wird heutzutage dem letztgenannten Gebiet der Wissenschaft zuteil, da die faszinierenden Eigenschaften der Materialien im Nanomaßstab neue Horizonte und Möglichkeiten bei der Diagnostik und der Behandlung von Krankheiten, wie zum Beispiel Krebs oder HIV, eröffnen.

Beispielsweise begannen Pharmazeuten bereits vor 70 Jahren mit der Einkapselung von Arzneimitteln im Inneren von Mizellen und Liposomen. Aufgrund der Entwicklungen auf dem Gebiet der Polymerchemie sind sie heutzutage in der Lage, künstliche Vesikel herzustellen, die das Arzneimittel direkt zu dem kranken Organ, Gewebe oder sogar zu der kranken Zelle transportieren. Dadurch kann die Zerstörung des Arzneimittels durch das Immunsystem, verhindert werden (K. Kataoka et al, Advanced Drug Delivery Reviews 64 (2012) 37-48). Aus diesem Grund haben Chemiker eine große Anzahl verschiedener biokompatibler Polymere entwickelt, die hervorragende Eigenschaften in Bezug auf Selbstassemblierung im Nanomaßstab haben. Durch diese Verfahren können nicht nur einfache Mizellen und Vesikel hergestellt werden, sondern ebenfalls Biopolymere, die die Natur nachahmen. Solche Materialien und Composites, die die Struktur von Pflanzen und Tieren kopieren, werden "bioinspirierte" oder "bionachahmende" Materialien genannt.

Ein typisches Beispiel eines solchen bioinspirierten Materials ist ein auf Abalonmuschelschalen-basierender Nanocomposite, der aufgrund seiner Architektur verbesserte mechanische Eigenschaften hat (P. Y. Chen et al, Progress in Materials Science 57 (2012) 1492-1704). Die Hauptidee hinter dieser Struktur ist die hierarchische Organisation von primären Materialbausteinen (im Falle der Muschel sind sie hauptsächlich durch verschiedene Calciumcarbonate, wie zum Beispiel Aragonit und Calcit dargestellt), welche in die Biopolymermatrix eingebunden sind. Eine solche Konstruktion der Muschelschale ermöglicht es der Molluske sich selbst vor Feinden und nachteiligen Bedingungen der Umgebung zu schützen. Die mechanischen und strukturellen Eigenschaften der Mollusken werden bereits gut verstanden und nun entwickeln die Forscher in der Chemie und der Materialwissenschaften künstliche Nanocomposites, um dieselben verbesserten Eigenschaften der neuen nanostrukturierten Composite zu erhalten.

Der vorliegenden Erfindung liegt die Aufgabe zugrunde, sich die Eigenschaften dieser nanostrukturierten Verbindungen bei der spezifischen Behandlung von Krankheiten zu Nutze zu machen. Dazu war die Aufgabe der vorliegenden Erfindung, Verbindungen (Nanocomposites) bereitzustellen, die spezifisch und selektiv Zellen (vollständig) einkapseln können, um dadurch zielgenau Krankheiten behandeln zu können. Diese Aufgabe konnte dadurch gelöst werden, dass Partikel (Mikro-, Meso- oder Nanoteilchen eines geeigneten Materials) mit Polymeren, welche wiederum mit spezifischen Antikörpern für die gezielte Erkrankung funktionalisiert sind, zu einem Polymer-Partikel-Komplex zu vereinen.

Daher betrifft ein Aspekt der vorliegenden Erfindung einen Polymer-Partikel-Komplex, der ein (Mikro-, Meso- oder Nano-) Partikel als Basiskomponente; eine Polymerhülle; und ein daran gebundenen Antikörper umfasst.

Ein weiterer Aspekt der vorliegenden Erfindung liegt in der Verwendung der erfindungsgemäßen Polymer-Partikel-Komplexe zur Einkapselung von Zellen.

Ein weiterer Aspekt der vorliegenden Erfindung liegt in der Verwendung der erfindungsgemäßen Polymer-Partikel-Komplexe bei der Behandlung von Krankheiten.
Figur 1 zeigt den prinzipiellen Aufbau eines erfindungsgemäßen Polymer-Partikel-Komplexes.
Figur 2 zeigt den Bindungsmechanismus des erfindungsgemäßen Polymer-Partikel-Komplexes an die erkrankte Zelle.

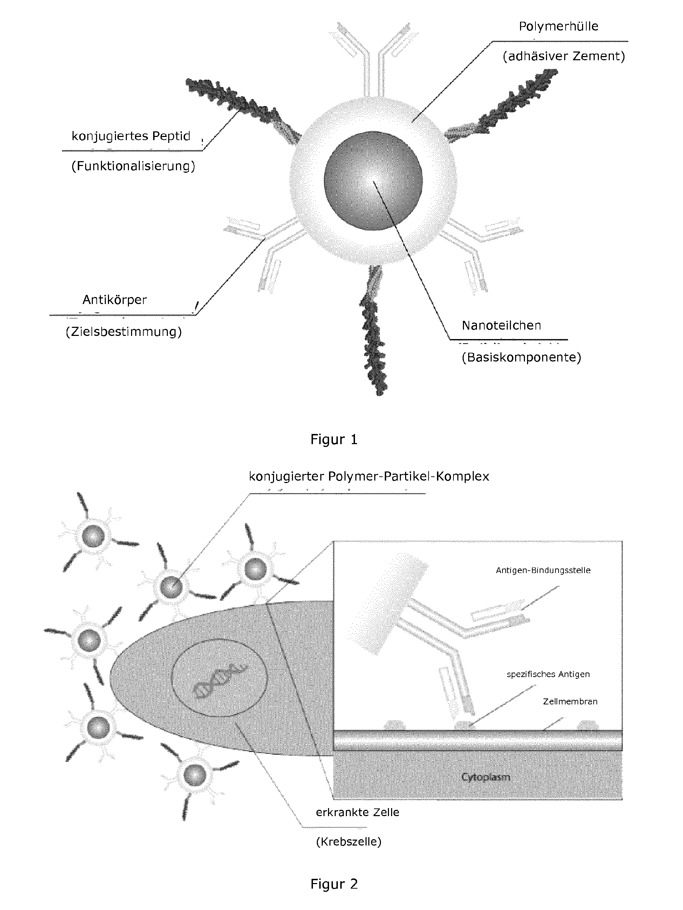

Der Polymer-Partikel-Komplex der vorliegenden Erfindung umfasst ein (Mikro-, Meso- oder Nano-) Partikel als Basiskomponente; eine mit dem Partikel verbundene Polymerhülle; und einen an die Polymerhülle gebundenen Antikörper. Figur 1 zeigt den generellen Aufbau eines solchen Komplexes, wobei der Partikel als die Basiskomponente den "Kern" bildet und als eine Art "Ziegelstein" eine äußere Wand oder Hülle um die betreffende Zelle bildet. Um die Basiskomponente herum befindet sich die Polymerhülle, die als eine Art "Zement" dient, der die "Ziegelsteine" und damit die Polymer-Partikel-Komplexe adhäsiv verbindet. An der Polymerhülle verankert befinden sich Antikörper, die zur Bestimmung und genauen Ansteuerung der Zielzelle nötig sind. Optional können noch Peptide an der Polymerhülle verankert sein.

Der (Mikro-, Meso- oder Nano-) Partikel, der als Basiskomponente dient, kann aus jedem für diesen Zweck geeigneten Material ausgewählt werden. Beispielsweise kann der Partikel ein magnetisches Teilchen, ein Biomineral, auf Kohlenstoff-basierende Nanostrukturen, Übergangsmetallverbindungen oder ein Alkalimetall und dessen Verbindungen sein, ohne darauf beschränkt zu sein. Der Partikel kann je nach Eigenschaft verschiedene unten beschriebene Charakteristika haben. In einer Ausführungsform der vorliegenden Erfindung ist die Basiskomponente ein Nanopartikel. In einer weiteren Ausführungsform der vorliegenden Erfindung ist die Basiskomponente ein Mesopartikel. In einer noch weiteren Ausführungsform der vorliegenden Erfindung ist die Basiskomponente ein Mikropartikel.

Magnetische Teilchen sind beispielsweise geeignet aufgrund ihrer Eigenschaft als Kontrastmittel für MRT-Untersuchungen und der thermischen Behandlung von Krebs durch die angewandten starken externen magnetischen Felder. Derartige Teilchen sind robust und stabil und es besteht die Möglichkeit, die wachsende, eingekapselte Hülle oder Schale über MRT-Kontrolle zu untersuchen und zu verfolgen. Beispiele solcher magnetischen Teilchen sind, ohne darauf beschränkt zu sein, Fe₂O₃, FeO, Fe₃O₄, Mn-doped FeO, Fe(CO)₅ oder MgO.

Biomineralien basieren auf den (Mikro- und Nano-)Strukturen, die es bereits in der Natur gibt, einschließlich Knochen, Muschelschalen, Hörnern oder Schnäbel. Diese Materialien besitzen gesteigerte mechanische Eigenschaften aufgrund ihrer Struktur im Nanomaßstab. Diese Materialien sind bereits seit Millionen von Jahren im Einsatz und haben sich im Laufe der Evolution bewährt. Zudem handelt es sich dabei um leichtgewichtige Strukturen. Beispiele solcher Biomineralien sind in der breiten Klasse von Verbindungen zu finden, die hauptsächlich auf Ca und Si basieren, wie zum Beispiel, ohne darauf beschränkt zu sein, Calcite, Aragonite, SiO₂, amorphe Kieselsäure usw.

Alkalimetalle und ihre Verbindungen haben aufgrund der Toxizität dieser Materialien für die Zellen eine erhöhte Heilungseffizienz. Beispielsweise nehmen die äußerst aggressiven Krebszellen derartige Partikel in das Zytoplasma auf, wo die Teilchen die Zellen vergiften können/werden. Beispielsweise führt die Freisetzungsenergie bei der Reaktion mit Wasser (70% der Zelle) bei reinen Alkalimetallen, welche in das Zytoplasma aufgenommen werden, zur Zellnekrose. Alkalimetalle und ihre Verbindungen haben eine leichtgewichtige Struktur und wasserfreie Salze, die Alkalimetallkationen besitzen, können als Trocknungsmittel verwendet werden: die gebildete Hülle oder Schale absorbiert Wasser von der Zelle, was zu deren Dehydrierung führt und folglich zu deren Tod. Beispiele für Alkalimetalle und ihre Verbindungen sind, ohne darauf beschränkt zu sein, Li, K, Na, LiHCO₃, NaHCO₃, KHCO₃, LiF, NaF, KF, LiCl, NaCl oder KCl. In einer Ausführungsform der Erfindung, wird das Alkalimetall oder seine Verbindungen aus Li, K, Na, NaHCO₃, LiF, NaF, oder KF ausgewählt. In einer noch weiteren Ausführungsform ist das Alkalimetall oder seine Verbindungen Li, Na, K oder NaHCO₃.

Übergangsmetallverbindungen basierend auf den Metallen Ti, Cr, Zn, Mn, Zr, Au, Pt, Pd, Ag und Cu können ebenfalls als Basiskomponente verwendet werden. Derartige Komplexe haben verschiedenste Eigenschaften, die die Charakteristika der zuvor beschriebenen Verbindungen kombinieren. Beispielsweise können Sauerstoffverbindungen dieser Metalle verwendet werden. Beispiele solcher Sauerstoffverbindungen sind, ohne darauf beschränkt zu sein, TiO₂, Cr₂O, ZnO, Mn₂O₃, oder ZrO.

Auf Kohlenstoff-basierende Nanostrukturen sind Kohlenstoff-Verbindungen, wie zum Beispiel, Kohlenstoffnanoröhrchen, Graphen oder Diamant.

In einer Ausführungsform der vorliegenden Erfindung wird die Basiskomponente aus der Gruppe, die aus Fe₂O₃, MgO, Kalziten, Aragoniten, Kieselsäure (SiO₂), Li, Na, K, NaHCO₃, NaCl, Kohlenstoffnanotubes und Graphen besteht, ausgewählt.

Der erfindungsgemäße Polymer-Partikel-Komplex umfasst neben dem oben genannten Partikel des Weiteren eine Polymerhülle oder -schale. Die Polymere werden aus der Gruppe, die nicht darauf beschränkt ist und die aus hydrophilen Polymeren, bioabbaubaren Polymeren, quellfähigen Polymeren, bioadhäsiven Polymeren, Ionenaustausch Polymeren, hydrophoben Polymeren, natürlichen Biopolymeren, Gerüstpolymeren für Gewebezüchtung und Kronenether besteht, ausgewählt. Beispiele für den erfindungsgemäßen Polymer-Partikel-Komplex verwendbare Polymere, die von der World Food and Drug Administration (WFDA)) genehmigt wurden, sind Poly(2-hydroxyethylmethacrylat), Poly(vinylpyrrolidon), Polymilchsäure, Polyglykolsäure, Collagen, Ethyl/Vinyl-Alkohol, Polycarbophil, Fibronektin, Polystyrol, Polystyrolsulfonsäure, Polydimethylsiloxan, Polyethylen, Polypropylen, Ethylen/Vinylacetat, Polyurethan, Polymethyl(meth)acrylat, Polyethylenterephthalat, Polyamide, Polytetrafluorethylen oder Polyvinylchlorid. Beispiele für natürliche Biopolymere sind, ohne darauf beschränkt zu sein, Verbindungen, die beispielsweise Alanin, Glycin oder Cystein in ihrer Struktur besitzen, wie zum Beispiel Spinnenweben, Abalonmuschelschalen oder Glasschwämme. Beispiele für Gerüstpolymere für Gewebezüchtung sind, ohne darauf beschränkt zu sein, Collagen, Chitosan oder Keratin. Beispiele für Kronenether sind, ohne darauf beschränkt zu sein, 12-Krone-4, 15-Krone-5, 18-Krone-6, Dibenzo-18-Krone-6 oder Diaza-18-Krone-6. Zusätzlich zu den oben genannten Polymeren können ebenfalls OTS, Thiole, Lipide, Fettsäure usw.

Bei einer Ausführungsform der vorliegenden Erfindung werden bioabbaubare Polymere verwendet. Diese Polymere sind beispielsweise resorbierbar, d.h. sie können vom Körper nach der Nutzungsphase abgebaut und resorbiert werden. Wie oben bereits ausgeführt, sind verwendbare bioabbaubare Polymere beispielsweise Poly(milchsäure), Polycaprolacton, Stärke, Cellulose, Polyhydroxyalkanoate, oder Polytrimethylenterephthalat usw.

Generell ist die Auswahl der möglichen Polymere nicht beschränkt und der Durchschnittsfachmann kann anhand des gewünschten Einsatzgebiets das passende Polymer wählen. Die Polymere des erfindungsgemäßen Polymer-Partikel-Komplexes können leicht durch dem Durchschnittsfachmann bekannte, geeignete Verfahren an die Basiskomponente gebunden werden. Beispielsweise hängt die Auswahl des Polymers von den Eigenschaften der ausgewählten Basiskomponente ab, beispielsweise von deren mechanischen und chemischen Charakteristika. Die mechanischen Eigenschaften des resultierenden Nanocomposites können dann beispielsweise numerisch berechnet werden, um Vergleichsdaten zu erhalten, die es erleichtern, den wirksamsten Polymer-Partikel-Komplex auszuwählen.

Der erfindungsgemäße Polymer-Partikel-Komplex umfasst zudem einen gebundenen Antikörper. Der Antikörper ist dafür verantwortlich, dass der erfindungsgemäße Polymer-Partikel-Komplex exakt an ein spezifisches Antigen, das von der erkrankten, lebenden Zelle exprimiert wird, bindet. Dadurch ist es möglich, dass der erfindungsgemäße Polymer-Partikel-Komplex prinzipiell an jede erkrankte Zelle oder jede Zielzelle binden kann, damit dort die gewünschte Einkapselung stattfinden kann. Beispiele für Antikörper, exprimiert durch verschiedene Krebszellarten, können der folgenden Tabelle 1 entnommen werden, sind aber nicht darauf beschränkt (siehe R. A. Weinberg, The Biology of Cancer, Garlad Science, 2007).

**Tabelle 1:**

| **Menschlicher Tumor** | **Spezifisches Antigen** | **Antigenes Protein (Antikörper)** |
|---|---|---|
| Brustkarzinom | ETA; HER2/Neu oder MUC-1 | KIFGSLAFL |
| Dickdarmkarzinom | CEA | YLSGANLNL |
| Ovarialkarzinom | CA-125, HER2/Neu | KIFGSLAFL |
| Kopf- und Nackenkarzinom | caspase8 | FPSDWCVF |
| Chronische myeloische Leukämie | Bcr-Abl | ATGFKQSSKALQRRPVAS |
| Prostatakarzinom | PSMA | FLPTKKKLQCV, VISNDVCAQV |
| Melanom | MAGE oder tyronaise | EADPTGHSY, SAYGEPRKL, MLLAVLYCL, YMNGTMSQV |

Der erfindungsgemäße Polymer-Partikel-Komplex kann weiter ein gebundenes Peptid umfassen. Das gebundene Peptid kann verschiedene Aufgaben erfüllen, beispielsweise die Stabilisierung des entworfenen Komplexes und der Schutz vor Zerstörung durch das Immunsystem. Zudem kann das Peptid mit in die Zelle aufgenommen werden und dort die DNA der Zelle verändern, diese damit umprogrammieren. Dementsprechend ist die vorliegende Erfindung auf kein spezielles Peptid beschränkt, sondern der Durchschnittsfachmann kann das entsprechende Peptid fallspezifisch auswählen.

In einer Ausführungsform der vorliegenden Erfindung wird der erfindungsgemäße Polymer-Partikel-Komplex zur Einkapselung von Zellen verwendet.

Ohne auf eine spezielle Theorie gebunden zu sein, ist der prinzipielle Bildungsmechanismus der Einkapselungsreaktion in Figur 2 zu sehen. Beispielhaft wird das Verfahren im Folgenden an der Einkapselung von Krebszellen erläutert, ist aber auf jede andere Zelle analog anwendbar.

Der erfindungsgemäße Polymer-Partikel-Komplex wandert durch die Verwendung von konjugierten Antikörpern, die für eine spezielle Krebszellenart spezifisch sind, zu den entsprechenden Krebszellen und bindet nur an diese durch die sich bildende Antikörper-Antigen-Bindung. Beispielsweise, wenn ein Patient an einem Adenokarzinom der Prostata leidet, kann ein Prostata-spezifisches Membran-Antigen (PSMA), das von der Krebszelle exprimiert wird, als Anbindungsstelle für den entworfenen Polymer -Partikel-Komplex dienen, was sicherstellt, dass der erfindungsgemäße Polymer-Partikel-Komplex exakt zu dem Krebsgewebe oder der Zielzelle innerhalb des Blutstroms transportiert wird. Es ist ebenfalls wichtig zu erwähnen, dass das Krebsgewebe ein hochentwickeltes Blutgefäßsystem besitzt, da die schnell wachsenden Zellen mehr Sauerstoff und Nährstoffe benötigen. Deshalb werden alle erfindungsgemäßen Bausteine zu dem Krebsgewebe transportiert, um dort angelagert zu werden.

Wenn die erfindungsgemäße Polymer-Partikel-Komplexe bei den Ziel-Krebszellen ankommen, werden sie an die Zellmembran gebunden, wodurch sie Schicht um Schicht wachsen und dabei eine "Wand" oder "Hülle" um die Zelle herum bilden, bei der die Basiskomponente ein "Ziegelstein" ist, der als Grundbaustein zum Aufbau der "Hülle" dient, und das Polymer als adhäsiver "Zement" zum Zusammenhalt der sich bildenden Wand bzw. Hülle dient.

Ein Behandlungsverfahren, das den erfindungsgemäßen Polymer-Partikel-Komplex verwendet, hat gegenüber herkömmlichen Behandlungsverfahren mehrere Vorteile:
- die gebildete "Wand" oder "Hülle" hat gute mechanische Eigenschaften, wie zum Beispiel die einer Abalonmuschelschale, und begrenzt somit das Wachstum der Krebszellen;
- die eingekapselten Zellen werden ebenfalls vom Blutstrom isoliert, wobei sie dadurch vollständig von Sauerstoff- und Nährstoffzufuhr abgeschnitten werden;
- die erzeugte Nanocomposite-Hülle um das Krebsgewebe verhindert ebenfalls die Ausbreitung von Metastasen im Organismus.

Falls der Krebs das Stadium erreicht, bei dem Metastasen beginnen in anderen Organen zu wachsen, kann das erfindungsgemäße Verfahren ebenfalls angewendet werden. Wenn der Patient, der zum Beispiel ein Adenokarzinom in der Prostata hat, ebenfalls Lungenkrebs hat (welches das Organ ist, das am häufigsten von Metastasen von dieser Art von Krebs wird befallen wird), wird der erfindungsgemäße Polymer-Partikel-Komplex ebenfalls das Krebsgewebe in den Lungen einkapseln, da diese Zellen dieselben Antigene (PSMA) in ihren Membranen haben (dies ist ein Möglichkeit, wie eine Biopsie durchgeführt wird, wenn das entnommene Gewebe untersucht wird, um den Primärtumor zu finden). Generell ist es für dieses Verfahren von Bedeutung, dass die Krebszellen bei Zweittumoren (Metastasen) dieselben Antigene exprimieren wie der Primärtumor und somit der erfindungsgemäße Polymer-Partikel-Komplex die jeweiligen erkrankten Zellen im Körper bindet und einkapselt. Deshalb hat das erfindungsgemäße Verfahren ein großes Potential für die Behandlung von Krebs im Spätstadium.

Wie oben beschrieben kann jedes genannte Material bei der Einkapselungsreaktion verwendet werden und als Baustein für diese Strategie verwendet werden. Beispielsweise kann man einfach die Abalonmuschelschale kopieren, indem man Calciumcarbonat-Partikel verwendet. In einer anderen Ausführungsform können magnetische Nanoteilchen angewendet werden, um die Bildung der isolierenden Wand um das Gewebe über MRT zu kontrollieren (Eisenoxid-Partikel werden intensiv untersucht als Kontrastmittel für dieses Bildgebungsverfahren).

In einer Ausführungsform der vorliegenden Erfindung kann die Wirksamkeit des erfindungsgemäßen Verfahrens signifikant gesteigert werden, wenn zellfeindliche Materialien verwendet werden. Im Gegensatz zu den herkömmlichen Behandlungsstrategien, einschließlich Chemotherapie und Strahlentherapie, kann der aggressive Polymer-Partikel-Komplex die Krebszellen lokal töten, während der Rest des Organismus minimal unbeschädigt bleibt. Zusätzlich können als "Zement" langfristig bioabbaubare Polymere verwendet werden, welche im Körper einige Zeit nach der Behandlung zerstört werden.

Die Einkapselungsreaktion kann zur Einkapselung von jeglicher Art von Zellen verwendet werden. Voraussetzung ist, dass die entsprechende Zelle(n) direkt über eine Antikörper-Antigen-Wechselwirkung ansteuerbar ist/sind.

Aufgrund der hohen Spezifizität der Einkapselungsreaktion kann das Verfahren zur Behandlung einer Vielzahl von Erkrankungen verwendet werden. Der erfindungsgemäßen Polymer-Partikel-Komplex (Nanocomposite) kann abhängig von der Exprimierung von Antigenen des Krankheitserregers oder der befallenen Zelle andocken und die erfindungsgemäße Hülle aufbauen.

Beispiele für mögliche zu behandelnde Erkrankungen sind, ohne darauf beschränkt zu sein, Krebs, Infektionen, HIV, virale Erkrankungen, bakterielle Erkrankungen, Hepatitis oder Pilzinfektionen. In einer Ausführungsform ist die zu behandelnde Krankheit Krebs.

In einer Ausführungsform ist es möglich, zunächst eine einzelne Zelle einzukapseln, da die Krebszellen innerhalb des Gewebes sehr dicht wachsen. Es ist ebenfalls möglich ein Lymphoma oder eine Leukämiezelle mit den erfindungsgemäßen Verbindungen einzukapseln. Das erfindungsgemäße Verfahren kann ebenfalls beispielsweise bei der Behandlung von HIV-infizierten Lymphozyten angewendet werden, wenn das Nanocomposite selektiv um die Virus-infizierten Zellen gebildet wird. In einer weiteren Ausführungsform der vorliegenden Erfindung kann das gesamte erkrankte Gewebe eingekapselt werden, wenn der Polymer-Partikel-Komplex das komplette kanzerogene Gewebe von dem Gesunden isoliert.

Aus der vorgenannten Beschreibung wird ersichtlich, dass Modifikationen und Variationen der beschriebenen Erfindung möglich sind und diese Ausführungsformen ebenfalls innerhalb des Schutzumfangs der vorliegenden Erfindung liegen.

## Patentansprüche

1. Ein Polymer-Partikel-Komplex, der umfasst:
- ein Partikel als Basiskomponente;
- eine mit dem Partikel verbundene Polymerhülle; und
- ein an die Polymerhülle gebundener Antikörper.

2. Der Polymer-Partikel-Komplex nach Anspruch 1, der weiter ein an die Polymerhülle gebundenes Peptid umfasst.

3. Der Polymer-Partikel-Komplex nach einem der vorangegangenen Ansprüche, wobei die Basiskomponente ein magnetisches Teilchen, ein Biomineral, auf Kohlenstoffbasierende Nanostrukturen, Übergangsmetallverbindungen oder ein Alkalimetall und dessen Verbindungen ist.

4. Der Polymer-Partikel-Komplex nach Anspruch 3, wobei die Basiskomponente aus der Gruppe, die aus Fe₂O₃, MgO, Kalziten, Aragoniten, amorphe Kieselsäure, SiO₂, Li, Na, K, NaHCO₃, NaCl, Kohlenstoffnanotubes und Graphen besteht, ausgewählt wird.

5. Der Polymer-Partikel-Komplex nach einem der vorangegangenen Ansprüche, wobei die Polymerhülle aus der Gruppe, die aus hydrophilen Polymeren, bioabbaubaren Polymeren, quellfähigen Polymeren, bioadhäsiven Polymeren, Ionenaustausch Polymeren, hydrophoben Polymeren, natürlichen Biopolymeren, Gerüstpolymeren für Gewebezüchtung und Kronenether besteht, ausgewählt wird.

6. Der Polymer-Partikel-Komplex nach einem der vorangegangenen Ansprüche zur Verwendung zur Einkapselung von Zellen.

7. Der Polymer-Partikel-Komplex nach Anspruch 6, wobei die Zellen lebende Zellen sind.

8. Der Polymer-Partikel-Komplex nach Anspruch 7, wobei die Zellen Krebszellen sind.

9. Der Polymer-Partikel-Komplex nach einem der Ansprüche 1 bis 5 zur Verwendung bei der Behandlung von Krankheiten.

10. Der Polymer-Partikel-Komplex nach Anspruch 9, wobei die Krankheit Krebs ist.
